# FASCICULE DE BREVET EUROPEEN

(11) **EP 2 627 645 B1**
(45) Date de publication et mention de la délivrance du brevet: **25.02.2015**
(21) Numéro de dépôt: 11785721.9
(22) Date de dépôt: 17.10.2011
(51) Int. Cl.: C07D 307/33, C07C 29/09

(54) **PROCEDE DE PREPARATION DE LA 2-HYDROXYBUTYROLACTONE**
VERFAHREN ZUR HERSTELLUNG VON 2-HYDROXYBUTYROLACTON
PROCESS FOR THE PREPARATION OF 2-HYDROXYBUTYROLACTONE

(30) Priorité: 15.10.2010 FR 1058440
(43) Date de publication de la demande: 21.08.2013
(73) Titulaire: Adisseo France S.A.S., 92160 Antony (FR)
(72) Inventeur: MONBRUN, Jérôme, F-38200 Chuzelles (FR); HENRYON, Vivien, F-69007 Lyon (FR)
(74) Mandataire: Delorme, Nicolas
(86) Numéro de dépôt international: PCT/FR2011/052417
(87) Numéro de publication internationale: WO 2012/049435

(56) Documents cités:
- WO-A1-2008/022953
- FR-A1- 2 150 605
- CHRISTIAN DAREMON ET RENÉ RAMBAUD: "Obtention et étude de quelques gamma-butanolides alpha-substitués (1er mémoire)", BULLETIN DE LA SOCIETE CHIMIQUE DE FRANCE, SOCIETE FRANCAISE DE CHIMIE. PARIS, FRANCE, no. 1, 1971, pages 294-301, XP009046464, ISSN: 0037-8968 cité dans la demande

## Description

La présente invention concerne un procédé de préparation de la 2-hydroxybutyrolactone (2HBL) à partir de l'acide 2-hydroxy-4-méthylthiobutyrique (abrégé indifféremment HMTBA, HMBA, AT88 ou Rhodimet AT88), de son analogue oxo, l'acide 2-oxo-4-méthylthiobutyrique (abrégé KMB), du 2-hydroxy-4-méthylthiobutyronitrile, ainsi que de leurs dérivés.

La 2HBL constitue un intermédiaire de synthèse important. Elle peut être préparée industriellement, de manière connue, à partir de l'acide malique en trois étapes (DE19735575A1, AU2004200948A), ou à partir de la γ-butyrolactone en deux étapes (WO2008/022953A1, Bull.Soc.Chim.Fr. 1971, 1, 294-301).

Le problème posé par ces deux voies réside d'une part, dans la difficulté à isoler la 2HBL et d'autre part, dans une production très importante de sels qu'il faut ensuite éliminer. La première voie de synthèse présente en outre les inconvénients d'engager des réactifs chers, à savoir BH₃ et TFAA ; l'utilisation de borane nécessite plus particulièrement des conditions de sécurité spécifiques. La seconde voie d'accès utilise des réactifs toxiques, à savoir Br₂ et PBr₃ et les performances décrites selon cette stratégie sont peu élevées (RR=23-52%). Globalement, ces deux approches restent très coûteuses à l'échelle industrielle et peu productives.

Les auteurs de la présente invention ont cherché à développer un procédé de synthèse de la 2HBL ne présentant pas ces inconvénients tout en restant dans un procédé simple, peu coûteux et efficace.

Le HMTBA est un analogue de la méthionine, acide aminé essentiel, et trouve des applications largement étendues notamment chez l'homme en tant que complément alimentaire ou médicament, ainsi qu'en nutrition animale, comme source de méthionine biodisponible. Les dérivés de cet analogue, notamment ses esters et ses sels, sont aussi utilisés dans les mêmes indications, certains d'entre eux, comme l'ester isopropylique du HMBA, présentant des propriétés supérieures à celles du HMBA. Le HMBA est produit à l'échelle industrielle selon des procédés parfaitement rodés, à raison de plusieurs centaines de milliers de tonnes/an. Son utilisation comme substrat de synthèse lui ouvre ainsi un avenir supplémentaire.

C'est dans ce contexte que les auteurs de la présente invention ont élaboré un procédé de synthèse de la 2HBL à partir du HMTBA et ses dérivés, qui, par rapport aux méthodes de synthèse précitées, peut être mis en oeuvre en quantités industrielles. Le procédé mis au point est réalisé en au plus trois étapes, dont les conditions de réaction sont souples et qui sont caractérisées par des taux de transformation élevés.

Il présente en outre les avantages de conduire à une 2HBL facilement isolable et purifiable, et ne produit par de sels en excès.

Ce procédé constitue donc une véritable solution à la synthèse industrielle de 2HBL, en levant l'ensemble des obstacles auxquels se heurtent les méthodes connues précitées.

Ainsi, un premier objet de l'invention est un procédé de préparation de la 2HBL à partir d'un composé, ou de son sel ou de ses oligomères, ledit composé répondant à la formule (I)

CH₃-S-CH₂CH₂CR1R2R3

Dans laquelle
R1 représente H
R2 représente un groupement choisi parmi OH ; OR4 et OCOR4 où R4 représente un groupement choisi parmi les groupements alkyle ayant de 1 à 10 atomes de carbone, linéaires, cycliques ou ramifiés, et les groupements aryle ayant de 6 à 10 atomes de carbone, éventuellement substitués par un ou des substituants choisis parmi les groupements alkyle ayant de 1 à 10 atomes de carbone, linéaires ou ramifiés, les halogènes et les groupements hydroxyle, amino, nitro et alcoxy ayant de 1 à 10 atomes de carbone; et OSiRR'R" où R, R' et R" sont choisis, indépendamment les uns des autres, parmi les groupements alkyle ayant de 1 à 10 atomes de carbone, linéaires, cycliques ou ramifiés, les groupements aryle ayant de 6 à 10 atomes de carbone, éventuellement substitués par un ou des substituants choisis parmi les groupements alkyle ayant de 1 à 10 atomes de carbone, linéaires ou ramifiés, ou R1 et R2 représentent ensemble =O,
R3 représente COOH ou un groupement COOR5 où R5 représente un groupement choisi parmi les groupements alkyle ayant de 1 à 10 atomes de carbone, linéaires, cycliques ou ramifiés, benzyle, et les groupements benzyle substitués par un ou deux substituants choisis parmi les groupements alkyle ayant de 1 à 10 atomes de carbone, linéaires ou ramifiés, les halogènes et les groupements hydroxyle, amino, nitro et alcoxy ayant de 1 à 10 atomes de carbone, ou R3 représente un groupe cyano,
   procédé selon lequel

On obtient un sulfonium dudit composé, ledit sulfonium répondant à la formule (II)

[CH₃][CH₂CH₂CR1R2R3][CR6R7R8]S⁺ X⁻

par réaction avec un agent de formule [CR6R7R8]X ou [CR6R7R8]⁺ X⁻,

Formules dans lesquelles R1, R2 et R3 ont la définition ci-dessus, et R6 et R7 sont choisis, indépendamment les uns des autres, parmi H, les groupements alkyle ayant de 1 à 10 atomes de carbone, linéaires, cycliques ou ramifiés, et les groupements aryle ayant de 6 à 10 atomes de carbone, éventuellement substitués par un ou des substituants choisis parmi les groupements alkyle ayant de 1 à 6 atomes de carbone, linéaires ou ramifiés, les halogènes et les groupements hydroxyle, amino, nitro et alcoxy ayant de 1 à 10 atomes de carbone ; R8 est choisi parmi H, les groupements alkyle ayant de 1 à 10 atomes de carbone, linéaires, cycliques ou ramifiés, les groupes aryle ayant de 6 à 10 atomes de carbone, éventuellement substitués par un ou des substituants choisis parmi les groupements alkyle ayant de 1 à 10 atomes de carbone, linéaires ou ramifiés, et les groupements attracteurs notamment ceux comprenant une fonction choisie parmi les fonctions acide, ester, cyano, et X représente un contre-ion, et

On hydrolyse le sulfonium ainsi obtenu en acide 2,4-dihydroxybutyrique on son sel, et

On cyclise l'acide 2,4-dihydroxybutyrique ou son sel en 2-hydroxybutyrolactone.

Avant de décrire l'invention plus en détails, la définition de termes employés dans cette description et les revendications est ci-après fournie.

### Définitions

Par sel d'un composé de formule I, on entend tout composé de formule I dans lequel l'hydrogène du groupement carboxylique est remplacé par un métal, notamment un métal alcalin, un métal alcalin-terreux ou un métal de transition. Ce métal est préférentiellement choisi parmi Na, Ca, Mn, Mg, Cr. II peut être simple ou multiple. Ainsi un sel de calcium de l'acide 2-hydroxy-4-méthylthiobutanoïque peut être choisi parmi les sels de formule (HMTBA)ₙ Ca où n varie de 2 à 10. Cette notion de sel couvre bien entendu tous mélanges de sels rentrant dans la définition ci-dessus.

Par oligomère d'un composé de formule I, on entend tout oligomère et notamment dimère tel qu'il peut coexister, y compris à l'état de traces, avec ledit composé lorsque ce dernier n'est pas employé à l'état totalement purifié.

Par préparation de la 2-hydroxybutyrolactone (2HBL), on entend couvrir toutes les formes de la 2HBL, seules ou en mélanges, notamment ses stéréoisomères et ses tautomères. En fonction de la ou les formes recherchées, l'homme du métier sélectionnera la ou les formes correspondantes de l'acide de départ.

Dans le cadre de la présente invention :
- Un groupement alkyle désigne un radical monovalent, hydrocarboné, saturé, linéaire, cyclique ou ramifié. Comme indiqué, il a de 1 à 10 atomes de carbone, de préférence 1 à 6 atomes de carbone. A titre d'exemples, entrent dans cette définition les groupements méthyle, éthyle, n-propyle, isopropyle, n-butyle, isobutyle, tert-butyle, sec-butyle, pentyle, néopentyle, n-hexyle, cyclohexyle..., ;
- Un groupement aryle désigne un radical monovalent, hydrocarboné, aromatique. A titre d'exemples, entrent dans cette définition les groupements phényle, naphtyle. A titre d'exemple de groupement aryle substitué, on peut citer le groupement tolyle.
- Un groupement alcoxy désigne un radical O-alkyle, où le terme alkyle répond à la définition ci-dessus ;
- Un contre-ion X est une entité qui va assurer l'électroneutralité du sulfonium de formule (II).

Ce procédé comprend les étapes d'obtention du sulfonium, d'hydrolyse du sulfonium en 24DHBA, et de cyclisation du 24DHBA en 2HBL. Comme cela sera indiqué plus bas, ces étapes sont consécutives ou concomitantes, selon les réactifs employés.

La première étape consiste à obtenir une forme activée dudit composé ou de son sel, les auteurs ont découvert, de manière inattendue, que la forme sulfonium pouvait conduire à la formation de 2HBL comme indiqué ci-dessus, notamment dans les conditions qui seront plus loin décrites.

Les documents FR2150605A1 et DE2161991A1 décrivent la préparation du sulfonium d'un acide 2-hydroxy-4-alkylthiobutyrique et notamment de l'HMTBA, par action d'un halogénure d'alkyle, de préférence en excès, sur ledit acide, en présence d'eau, à une température comprise entre 10 et 100°C, puis isolement du sulfonium par extraction avec un alcool, après élimination de l'eau. Selon l'invention, le sulfonium peut être obtenu de cette manière ou par toute autre réaction dite d' « alkylation » appropriée sur le composé (I) ou son sel.

Pour cette première étape, le réactif est de préférence un agent de formule [CR6R7R8]X ou R6, R7 et R8 sont tels que définis ci-dessus et X est choisi parmi les halogènes et les groupements OH, sulfate, sulfonate et phosphate. Lorsque X désigne un halogène, un agent préféré pour la mise en oeuvre d'un procédé de l'invention à l'échelle industrielle est choisi parmi l'iodure de méthyle, l'acide bromoacétique et le bromure de benzyle. Lorsque X désigne OH, l'agent est choisi parmi les alcools ayant de 2 à 6 atomes de carbone, linéaires ou ramifié et est de préférence employé en milieu acide, par exemple en présence d'acide sulfurique ; un agent avantageux notamment eu égard à un procédé industriel, est le tertio-butanol. Dans le cas ou l'agent est un alcool et notamment le tertio-butanol, un milieu réactionnel favorable est un milieu hydroalcoolique acide.

Selon une autre variante, obtient le sulfonium par réaction sur le composé, d'un agent de formule [CR6R7R8]⁺X⁻ où [CR6R7R8]⁺ est un carbocation formé à partir d'un alcène correspondant, ayant de 2 à 10 atomes de carbone, linéaires ou ramifiés, en présence d'un acide. Cet acide sera choisi par l'homme du métier à partir de ses connaissances générales pour former le carbocation. C'est de préférence un acide minéral, par exemple l'acide sulfurique ou l'acide chlorhydrique.

A titre préféré, l'agent est de formule [C(CH₃)₂H]⁺X⁻ où X représente HSO₄ ou CI et est formé à partir de propène en présence d'acide sulfurique ou d'acide chlorhydrique, respectivement. Selon une autre variante avantageuse, l'agent est de formule [C(CH₃)₃]⁺X⁻ où X représente HSO₄ ou Cl et est formé à partir d'isobutène en présence d'acide sulfurique ou d'acide chlorhydrique, respectivement.

Tous autres agents conduisant à la formation du sulfonium peuvent bien entendu être employés. Par ailleurs, l'agent d'alkylation peut être supporté.

L'étape d'hydrolyse du sulfonium selon le procédé de l'invention peut être envisagée dans toutes conditions adaptées. A titre d'exemple, elle est réalisée par simple chauffage du milieu réactionnel directement issu de l'étape précédente d'alkylation. De préférence, le pH n'est pas trop élevé, avantageusement il est maintenu à une valeur de l'ordre de 6.

La dernière étape du procédé de l'invention est la cyclisation de 24DHB en 2HBL peut être réalisée par l'homme du métier dans des conditions décrites par exemple dans les documents AU2004200948A et WO2008/022953A1 précités.

Au moins deux, voire toutes les étapes de formation du sulfonium, d'hydrolyse du sulfonium en 24DHBA et de cyclisation du 24DHBA peuvent être simultanées. Des conditions de chauffage à une température variant de 30 à 150°C sont suffisantes, préférentiellement de 60 à 100°C. Les auteurs ont en outre observé que l'ajout de sels d'halogénure, tels que NaBr, permet d'augmenter la réactivité et la sélectivité de ces réactions.

Comme indiqué précédemment, la 2HBL ainsi formée peut être facilement purifiée et isolée du milieu réactionnel. Les exemples qui suivent l'illustreront, mais l'homme du métier aura recours à ses connaissances générales dans ce domaine, pour y procéder. Ainsi, toutes les techniques par décantation, distillation... bien connues peuvent être appliquées.

La présente invention et ses avantages sont illustrés dans les exemples qui suivent.

Dans la partie expérimentale ci-après, TT signifie taux de transformation, RR rendement sur réactif, SAAT88 sulfonium acétique de l'AT88, SBAT88 sulfonium benzylique de l'AT88, MHACa sel de calcium de l'AT88, SBMHACa sulfonium benzylique MHACa, T_{DE} et T_{MR} température de la double enveloppe et du milieu réactionnel respectivement, DCM dichlorométhane

### Exemple 1 : Préparation de la 2HBL à partir de l'HMTBA (ou AT88) par l'intermédiaire d'un sulfonium obtenu par réaction de l'acide bromoacétique

### 1.1. Préparation de la 24DHBA :

### Schéma réactionnel :

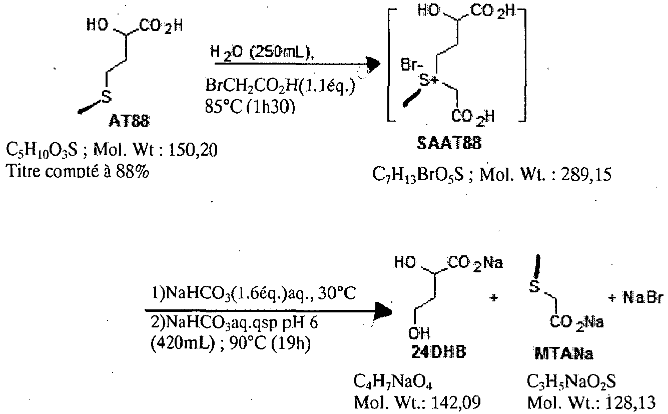

Réactifs et tableau des charges :

| Réactifs | Poids Moléculaire | Qualité % | Densité (g/ml) | Quantité (g) | Volume (ml) | mmoles | Eq. |
|---|---|---|---|---|---|---|---|
| AT88 | 150.2 | 88 | - | 50.0 | - | 293 | 1.0 |
| BrCH₂CO₂H | 138.9 | >98% | - | 45.4 | - | 326 | 1.1 |
| Eau | - | desionisée | 1.0 | 250 | 250 | - | - |
| NaHCO₃ | 84.0 | - | - | 39.1 | - | 466 | 1.6 |
| NaHCO₃aq. | 84.0 | 8.7% | - | *(36.5)* | 420 | 435 | 1.5 |

### Conditions opératoires et résultats :

### 1.1.1. Alkylation

Dans un réacteur double enveloppe de 500mL équipé d'un réfrigérant, d'un thermomètre et d'une agitation mécanique à quatre pales inclinées, sont introduits successivement à 20°C : 50g d'HMTBA et 200mL d'eau désionisée (T_{DE}=20°C). Agitation du milieu à 400tr/min (solution laiteuse) puis ajout en 5 minutes de l'acide bromoacétique (45.4g, 1.1éq., pas d'exothermie) et rinçage par 50mL d'eau désionisée. 8 minutes après ajout de BrCH₂CO₂H, obtention d'un milieu limpide orangé (agitation à 400tr/min), pas de nette exothermie (T_{MR}=20°C, T_{DE}=20°C 10 minutes après ajout de l'acide bromoacétique).

Chauffage du milieu sous agitation (400tr/min) jusqu'à 80-85°C (consigne T_{MR}=80°C atteinte en 30 minutes, T_{DE}=95°C).

Maintien du chauffage et de l'agitation durant 1h30 à 80-85°C (T_{DE}=80°C (30min) puis T_{DE}=85°C (1 h)). Obtention d'une solution limpide orangée.

Prélèvement pour analyse RMN¹H (100µL solution brute+500µL D₂O).

Critère d'arrêt : AT88 résiduel<1%mol (triplet δ=2.4ppm, 2H, D₂O) ⇒ résultat conforme.

### 1.1.2. Hydrolyse pH 6

Le brut réactionnel précédent est refroidi à 30°C sous agitation (300tr/min, consigne T_{MR}=30°C atteinte en 20 minutes, T_{DE}=20°C). Une sonde pH est introduite dans le même réacteur. Une fois le milieu à 30°C, ajout en 30 minutes par portions de NaHCO₃ solide (39g, 1.6éq.); forte effervescence « à retard ». En fin d'ajout, pH mesuré =3.1 à 25°C. Solution limpide orangée.

Chauffage du milieu à 90°C (consigne T_{MR}=90°C atteinte en 30 minutes, T_{DE}=95°C). Agitation à 400tr/min.

A T_{MR}=90°C (30 minutes après le début du chauffage ; pH relevé=3.0), la régulation du pH est démarrée avec la consigne pH=6 par ajout d'une solution aqueuse de NaHCO₃ 8.7% (via un pousse-seringue contrôlé par ordinateur).

Après 3h30 de régulation, la quantité de NaHCO₃ ajoutée est de 410mL (pH=6.0). L'agitation est ramenée à 100tr/min et le chauffage est maintenu durant toute la nuit (T_{DE}=95°C, T_{MR}=90°C).

Après 19h de régulation, le pH du milieu est de 6.1. Prélèvement de la phase aqueuse (100µL+500µL D₂O) pour analyse RMN¹H.

Critère d'arrêt : disparition du signal caractéristique du SAAT88 (singulet δ=2.81ppm et multiplet à δ=3.33ppm, D₂O) ⇒ résultat conforme (SAAT88 non détecté).

Retour à 25°C en 1h30 sous légère agitation.

Obtention de 748g de brut aqueux.

### Résultats :

- TT_{AT88}>99% (étape 1, estimé par RMN¹H)
- RR_{24DHB}=95% (dosé par RMN¹H)
- RR_{MTANa}=95% (dosé par RMN¹H)
- AT88 résiduel : <2%mol (estimé RMN¹H)

### 1.2 Synthèse et isolement de la 2HBL à partir de 24DHB

### Schéma réactionnel :

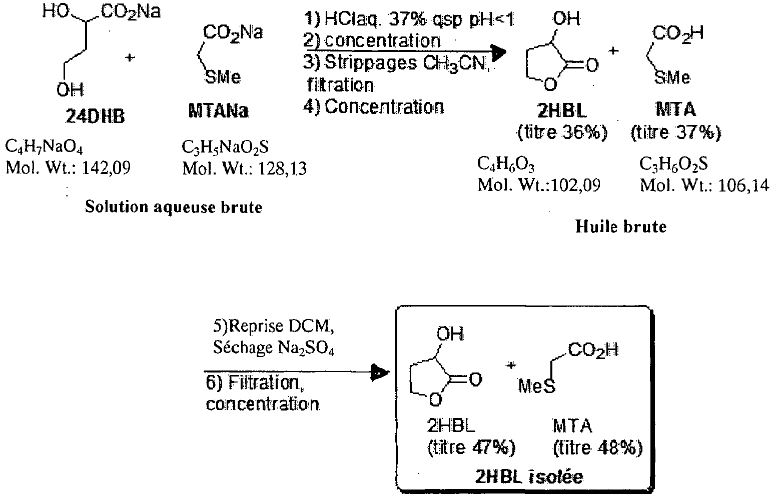

### Réactifs et tableau des charges :

| Réactifs | Poids Moléculaire | Qualité % | Densité (g/ml) | Quantité (g) | Volume (ml) | mmoles | Eq. |
|---|---|---|---|---|---|---|---|
| Brut aqueux | 142.1 | 0.37mmol/g | - | 74.8 | - | 27.8 | 1.0 |
| HClaq.37% | 36.5 | 37% | 1.2 | 7.2 | 6 | 73 | qsp pH<1 |
| Brut aqueux acidifié | 120.1 | 0.34mmol/g | - | 82 | - | 27.8 | 1.0 |
| CH₃CN | - | HPLC | | - | 2x100 +100 | - | - |
| Huile brute | 102.1 | - | - | 1.0 | - | | - |
| DCM | - | PA | 1.33 | - | 40 | - | - |
| Na₂SO₄ | - | Anhydre | - | 2.0 | - | - | - |

### Conditions opératoires et résultats :

### a) Acidification

Dans un ballon tricol de 250mL muni d'une agitation magnétique et équipé d'une électrode pH sont introduits 74.8g de la solution aqueuse de 24DHB puis HCl_{aq.} 37% est ajouté goutte à goutte jusqu'à pH=0.5 (ajout de 6mL). Solution orangée limpide.

### b) Concentration et strippages CH₃CN

La solution acidifiée précédemment préparée est introduite dans un ballon de 250mL et concentrée sous pression réduite (20mbar, 60°C). Le brut concentré (huile+solide) est repris par 100mL d'acétonitrile, puis la suspension obtenue est concentrée (60°C, 20mbar). Cette opération est renouvelée une fois puis 100mL d'acétonitrile sont ajoutés et la suspension obtenue est filtrée sur fritté de porosité N°3 ; les sels et insolubles sont rincés par 2x10mL d'acétonitrile puis le filtrat est concentré (17mbar, 60°C). Obtention de 6.6g d'huile jaune-orangé pâle.

### c) Séchage sur Na₂SO₄

Dans un tube Schott de 100mL muni d'une agitation magnétique 1.0g d'huile brute obtenue précédemment sont dissous par 40mL de dichlorométhane (solution trouble, laiteux et présence légère de résidu gommeux) puis 2g de Na₂SO₄ sont ajoutés sous agitation. Maintien de l'agitation durant 30 minutes puis filtration sur fritté de porosité N°3 (filtrat limpide) ; rinçage des sels par 40mL de DCM. Le filtrat est concentré sous pression réduite (19mbar, 35°C).

Obtention de 0.75g d'huile jaune pâle. Analyse RMN¹H (CDCl₃)

### Résultats (dosés par RMN¹H) :

- RR_{2HBL}=82% ; titre 2HBL = 47% (à partir de 24DHB)
- RR_{MTA}=81% ; titre MTA = 48% (à partir de MTANa)

### Exemple 2 : Préparation de la 2HBL à partir de l'HMTBA (ou AT88) par l'intermédiaire d'un sulfonium obtenu par réaction du bromure de benzyle

### 2.1. Préparation de la 24DHBA :

### Schéma réactionnel :

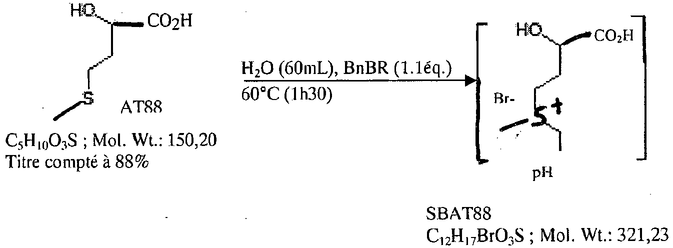

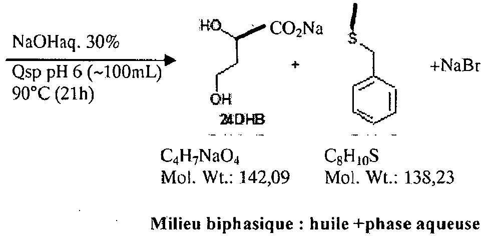

Réactifs et tableau des charges :

| Réactifs | Poids Moléculaire | Qualité % | Densité (g/ml) | Quantité (g) | Volume (ml) | mmoles | Eq. |
|---|---|---|---|---|---|---|---|
| AT88 | 150.2 | 88 | | 60 | - | 352 | 1.0 |
| BnBr | 171.0 | 100 | 1.438 | 66 | 46 | 383 | 1.09 |
| Eau | - | désionisée | 1.0 | 60 | 60 | - | - |
| NaOHaq. 30% | 40.0 | 7.5 N | - | - | 100 | 750 | 2.13 |

### Conditions opératoires et résultats :

### 1) Alkylation

Dans un réacteur double enveloppe de 250mL équipé d'un réfrigérant, d'un thermomètre et d'une agitation mécanique quadripale, sont introduits successivement à 25°C : 60g d'AT88 et 60mL d'eau désionisée (T_{DE}=25°C). Agitation du milieu à 500tr/min (émulsion beige) puis ajout en 3 minutes du bromure de benzyle (46mL, 1.1 éq.) ; milieu biphasique, agitation à 1000tr/min pour obtenir un milieu bien émulsionné (milieu laiteux marron clair). L'ajout de BnBr est exothermique (6 minutes après ajout BnBr : T_{MR}=36°C, T_{DE}=25°C).

Chauffage du milieu sous agitation (1000tr/min) jusqu'à 62°C (consigne T_{MR}=62°C atteinte en 30 minutes, T_{DE}=65°C).

Maintien du chauffage et de l'agitation durant 1h30 à 62°C (T_{DE}=65°C). Obtention d'une solution limpide orangée.

Prélèvement pour analyse RMN¹H (50µL solution brute+500µL D₂O).

Critère d'arrêt : disparition du signal caractéristique de l'AT88 (triplet δ=2.4ppm, D₂O) ⇒ résultat conforme (AT88 non détecté).

### 2) Hydrolyse pH 6

Une sonde pH est introduite dans le même réacteur. Chauffage du milieu à 90°C (consigne T_{MR}=91°C atteinte en 30 minutes, T_{DE}=100°C puis 93°C). Agitation à 700tr/min.

A T_{MR}=85°C (24 minutes après le début du chauffage ; pH relevé=-0.6), un prélèvement du milieu est effectué pour analyse RMN¹H (50µL+500µL D₂O) pour vérifier la formation d'AT88 avant début de régulation ⇒ présence d'AT88 confirmée (signaux caractéristiques : triplet δ=2.4ppm et singulet δ=1.87ppm).

La régulation du pH est démarrée après ce prélèvement avec la consigne pH=6 par ajout de soude 30% (via un pousse-seringue contrôlé par ordinateur). A pH=3.5, le milieu réactionnel se trouble et devient laiteux (3minutes de régulation). Après 7 minutes de régulation (pH=6.0, consigne atteinte), formation d'une huile orangée qui surnage. Après 1h de régulation, présence significative de surnageant huileux. Agitation à 400tr/min.

Après 5h de régulation, la quantité de soude ajoutée ne varie quasiment plus (pH=6.13). L'agitation est ramenée à 100tr/min et le chauffage est maintenu durant toute la nuit (T_{DE}=93°C, T_{MR}=90°C).

Après 19h de régulation, le pH du milieu est de 6,04. Prélèvement de la phase aqueuse (50µL+500µL D₂O) et de l'huile surnageante (30mg+600µL CDCl₃) pour analyse RMN¹H.

Critère d'arrêt : disparition du signal caractéristique du SBAT88 (singulet δ=2.58ppm, D₂O) ⇒ résultat conforme (SBAT88 non détecté).

Retour à 25°C en 3h sous légère agitation. Les deux phases sont séparées par simple décantation et soutirées.

Obtention de 142g d'huile brune et de 245g de brut aqueux.

### Résultats :

- TT_{AT88}=100% (étape 1, estimé par RMN¹H)
- RR_{24DHB}=83% (dosé par RMN¹H)
- RR_{AT88}=8% (dosé par RMN¹H)

### 2.2. Synthèse et isolement de la 2HBL à partir de 24DHB

### Schéma réactionnel :

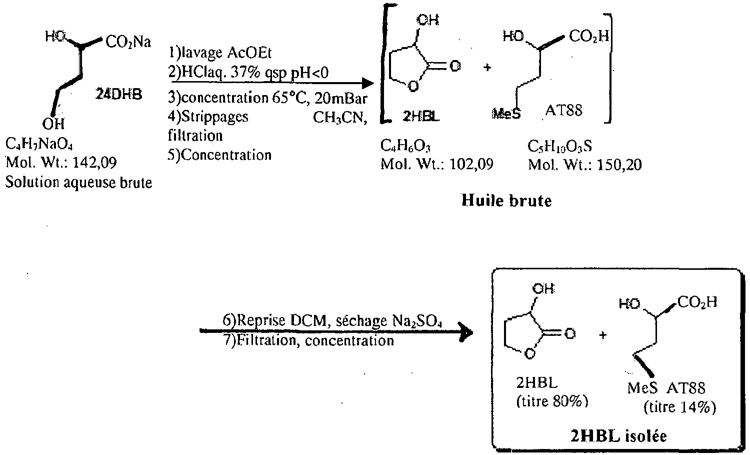

### Réactifs et tableau des charges :

| Réactifs | Poids Moléculaire | Qualité % | Densité (g/ml) | Quantité (g) | Volume (ml) | mmoles | Eq. |
|---|---|---|---|---|---|---|---|
| Brut aqueux | 142.1 | 1.2mmol/g | - | 50 | - | 59.6 | 1.0 |
| AcOEt | - | PA | | - | 3x15ml | - | - |
| HClaq. 37% | 36.5 | 37% | 1.2 | 7.2 | 6 | 73 | qsp pH |
| Brut aqueux lavé acidifié | 120.1 | 1.2mol/g | - | 12.6 | - | 14.6 | 1.0 |
| CH₃CN | - | HPLC | | - | 3x50+50 | - | |
| Huile brute | 102.1 | - | - | 1.735 | - | - | - |
| DCM | - | PA | 1.33 | - | 50 | - | - |
| Na₂SO₄ | - | Anhydre | - | 2.0 | - | - | - |

### Conditions opératoires et résultats :

### a) lavages par AcOEt

Dans un tube schott de 100ml, sont introduits successivement 50g de phase aqueuse brute isolée par simple décantation du brut réactionnel d'hydrolyse puis 15mL d'acétate d'éthyle.

Agitation vigoureuse et décantation. La phase organique est éliminée. Le lavage par AcOEt de la phase aqueuse est reproduit deux fois.

Obtention de 49.1 g de brut aqueux, solution orangée limpide. Analyse RMN¹H (100µL+500µD₂O). Critère d'arrêt : résidus benzyliques <1%molaire évalués par comparaison du signal caractéristique benzylique (massif δ=7.3ppm, D₂O) au 24DHB (signal caractéristique δ=3.62ppm) ⇒ résultat conforme (résidus benzylés<1%mol).

### b) Acidification

Dans un ballon tricol de 100mL muni d'une agitation magnétique et équipé d'une électrode pH sont introduits 49g de la solution aqueuse précédente puis HClaq. 37% est ajouté goutte à goutte jusqu'à pH=-0.5 (ajout de 6mL). Solution orangée limpide.

### c) Concentration et strippages CH₃CN

12.3g de la solution acidifiée sont introduits dans un ballon de 100mL et concentrés sous pression réduite (20mbar, 65°C). Le brut concentré (huile+solide) est repris par 50mL d'acétonitrile, puis la suspension obtenue est concentrée (65°C, 20mbar). Cette opération est renouvelée trois fois puis 50mL d'acétonitrile sont ajoutés et la suspension obtenue est filtrée sur fritté de porosité N°3 ; les sels et insolubles sont rincés par 2x5mL d'acétonitrile puis le filtrat est concentré (20mbar, 65°C). Obtention de 1.73g d'huile jaune-orangé pâle.

### d) Séchage sur Na₂SO₄

Dans un ballon tricol de 100mL muni d'une agitation magnétique 1.73g d'huile brute obtenue précédemment sont dissouts par 50mL de dichlorométhane (solution trouble, formation légère de flocons blanc-cassé qui décantent) puis 2g de Na₂SO₄ sont ajoutés sous agitation. Maintien de l'agitation durant 30 minutes puis filtration sur fritté de porosité N°3 (filtrat légèrement trouble) ; rinçage des sels par 2x25mL de DCM. Le filtrat est concentré sous pression réduite (18mbar, 35°C).

Obtention de 1.52g d'huile jaune pâle. Analyse RMN¹H (CDCl₃)

### Résultats (dosés par RMN¹H) :

- RR_{2HBL}=81%; titre 2HBL = 80%
- RR_{AT88}=8%; titre AT88 = 14%

### Exemple 3 : Préparation de la 2BHL à partir d'un sel de calcium de l'HMTBA par l'intermédiaire d'un sulfonium

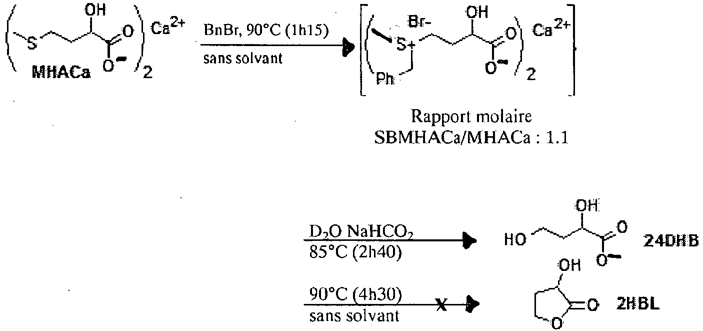

On n'observe pas la formation de 2HBL (ou formes ouvertes dérivées) même après chauffage prolongé à 90°C (4h30) du brut d'alkylation. A l'opposé, l'effet d'un ajout d'eau et d'hydrogénocarbonate de sodium sur le brut d'alkylation (qsp pH 8) suivi d'un chauffage s'est traduit par la formation significative de 24DHB. Dans les conditions testées, la présence d'eau est donc nécessaire au déplacement du sulfonium.

### Exemple 4 : Préparation de la 2BHL « one pot » et effet d'un rajout de sel (NaBr)

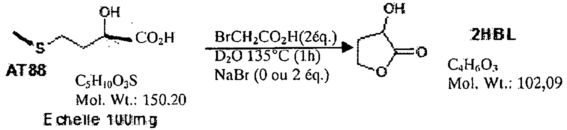

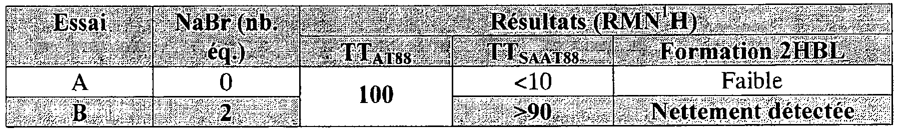

Les conditions utilisées pour l'essai B ont été reproduites avec les performances suivantes (dosage RMN¹H, après 3h à 135°C): TT_{SAAT88} complet, RR_{2HBL}=33-39%.

### Exemple 5 : Préparation du sulfonium méthylique du HMBA

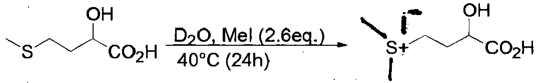

Conditions opératoires : mini-réacteur de 5mL ; à 25°C, introduction d'AT88 (644mg), D₂O (3mL), Mel (478µL) puis chauffage à 40°C pendant 24h. Concentration de la totalité du milieu réactionnel (18mbar, 65°C).
Résultats (dosés RMN¹H): TT AT88>95%, RRisolé (sulfonium) =89%, titre (sulfonium)=70%

### Exemple 6 : Préparation du sulfonium tert-butylique du HMBA

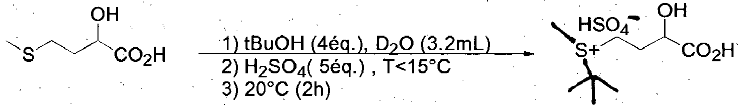

Conditions opératoires: Tube Schott de 30ml; à 25°C, introduction d'AT88 (3.4g), D₂O (3.2mL), tBuOH (7.64mL) puis refroidissement à 10°C et ajout en 20 minutes de H₂SO₄ (5,2mL, 5éq.) en maintenant une température T<15°C ; retour à 20°C après fin de l'ajout d'acide et maintien 2h à 20°C. Résultats (estimés RMN¹H) : TT AT88 complet, RRdosé (sulfonium) >90%

## Revendications

1. Procédé de préparation de la 2-hydroxybutyrolactone (2HBL) à partir d'un composé, ou de son sel ou de ses oligomères, ledit composé répondant à la formule (I)
CH₃-S-CH₂CH₂CR1R2R3
Dans laquelle
R1 représente H
R2 représente un groupement choisi parmi OH ; OR4 et OCOR4 où R4 représente un groupement choisi parmi les groupements alkyle ayant de 1 à 10 atomes de carbone, linéaires, cycliques ou ramifiés, et les groupements aryle ayant de 6 à 10 atomes de carbone, éventuellement substitués par un ou des substituants choisis parmi les groupements alkyle ayant de 1 à 10 atomes de carbone, linéaires ou ramifiés, les halogènes et les groupements hydroxyle, amino, nitro et alcoxy ayant de 1 à 10 atomes de carbone ; et OSiRR'R" où R, R' et R" sont choisis indépendamment les uns des autres parmi les groupements alkyle ayant de 1 à 10 atomes de carbone, linéaires, cycliques ou ramifiés, les groupements aryle ayant de 6 à 10 atomes de carbone, éventuellement substitués par un ou des substituants choisis parmi les groupements alkyle ayant de 1 à 10 atomes de carbone, linéaires ou ramifiés, ou R1 et R2 représentent ensemble =O,
R3 représente COOH ou un groupement COOR5 où R5 représente un groupement choisi parmi les groupements alkyle ayant de 1 à 10 atomes de carbone, linéaires, cycliques ou ramifiés, benzyle, et les groupements benzyle substitués par un ou deux substituants choisis parmi les groupements alkyle ayant de 1 à 10 atomes de carbone, linéaires ou ramifiés, les halogènes et les groupements hydroxyle, amino, nitro et alcoxy ayant de 1 à 10 atomes de carbone, ou R3 représente un groupe cyano,
procédé selon lequel
On obtient un sulfonium dudit composé, ledit sulfonium répondant à la formule (II)
[CH₃][CH₂CH₂CR1R2R3][CR6R7R8]S⁺X⁻
Par réaction avec un agent de formule [CR6R7R8]X ou de formule [CR6R7R8]⁺X⁻,
Formules dans lesquelles R1, R2 et R3 ont la définition ci-dessus, et R6 et R7 sont choisis indépendamment les uns des autres parmi H, les groupements alkyle, ayant de 1 à 10 atomes de carbone, linéaires, cycliques ou ramifiés, et les groupements aryle ayant de 6 à 10 atomes de carbone, éventuellement substitués par un ou des substituants choisis parmi les groupements alkyle ayant de 1 à 10 atomes de carbone, linéaires ou ramifiés, les halogènes et les groupements hydroxyle, amino, nitro et alcoxy ayant de 1 à 10 atomes de carbone ; R8 est choisi parmi H, les groupements alkyle ayant de 1 à 10 atomes de carbone, linéaires, cycliques ou ramifiés, les groupements aryle ayant de 6 à 10 atomes de carbone, éventuellement substitués par un ou des substituants choisis parmi les groupements alkyle ayant de 1 à 10 atomes de carbone, linéaires ou ramifiés, et les groupements attracteurs notamment ceux comprenant une fonction choisie parmi les fonctions acide, ester, cyano, et X représente un contre-ion, et
On hydrolyse le sulfonium ainsi obtenu en acide 2,4-dihydroxybutyrique ou son sel, et
On cyclise l'acide 2,4-dihydroxybutyrique ou son sel en 2-hydroxybutyrolactone.

2. Procédé selon la revendication 1, **caractérisé en ce que** le composé de formule (I) est choisi parmi l'acide 2-hydroxy-4-méthylthiobutyrique (HMTB), l'acide 2-oxo-4-méthylthiobutyrique (KMB), l'ester isopropylique du HMTB (HMBI), leurs sels et leurs oligomères.

3. Procédé selon la revendication 1 ou 2, **caractérisé en ce que** R2 représente OH, R6 et R7 sont indépendamment choisis parmi H et CH₃ et R8 est choisi parmi H et les groupements CH₃, phényle et COOH.

4. Procédé selon l'une quelconque des revendications 1 à 3, **caractérisé en ce qu'**on obtient le sulfonium par réaction sur le composé, d'un agent choisi parmi les agents de formule [CR6R7R8]X où X est choisi parmi les halogènes et les groupements OH, sulfate, sulfonate et phosphate.

5. Procédé selon la revendication 4, **caractérisé en ce que** ledit agent est choisi parmi l'acide bromoacétique et le bromure de benzyle.

6. Procédé selon la revendication 4, **caractérisé en ce que** ledit agent est choisi parmi les alcools ayant de 2 à 6 atomes de carbone, linéaires ou ramifiés, et est utilisé en milieu acide.

7. Procédé selon la revendication 1, **caractérisé en ce qu'**on obtient le sulfonium par réaction sur le composé, d'un agent de formule [CR6R7R8]⁺X⁻ où [CR6R7R8]⁺ est un carbocation formé à partir d'un alcène correspondant, ayant de 2 à 10 atomes de carbone, linéaires ou ramifiés, en présence d'un acide.

8. Procédé selon la revendication 7, **caractérisé en ce que** l'agent est de formule [C(CH₃)₂H]⁺X⁻ où X représente HSO₄ ou Cl et est formé à partir de propène en présence d'acide sulfurique ou d'acide chlorhydrique, respectivement.

9. Procédé selon la revendication 7, **caractérisé en ce que** l'agent est de formule [C(CH₃)₃]⁺X⁻ où X représente HSO₄ ou Cl et est formé à partir d'isobutène en présence d'acide sulfurique ou d'acide chlorhydrique, respectivement.

10. Procédé selon la revendication 1, **caractérisé en ce que**, au moins deux des réactions de formation du sulfonium, d'hydrolyse du sulfonium et de cyclisation de l'acide 2,4-dihydroxybutyrique (24DHBA) sont simultanées.

11. Procédé selon la revendication 10, **caractérisé en ce que** la température de réaction varie de 30 à 150°C.

12. Procédé selon la revendication 10 ou 11, **caractérisé en ce qu'**on ajoute des sels d'halogénure, comme NaBr.

13. Procédé selon l'une quelconque des revendications 1 à 12, **caractérisé en ce qu'**il est mis en oeuvre à l'échelle industrielle.

14. Utilisation d'un sulfonium répondant à la formule (II)
[CH₃][CH₂CH₂CR1R2R3][CR6R7R8]S⁺ X⁻
Dans laquelle
R1 représente H
R2 représente un groupement choisi parmi OH ; OR4 et OCOR4 où R4 représente un groupement choisi parmi les groupements alkyle ayant de 1 à 10 atomes de carbone, linéaires, cycliques ou ramifiés, et les groupements aryle ayant de 6 à 10 atomes de carbone, éventuellement substitués par un ou des substituants choisis parmi les groupements alkyle ayant de 1 à 10 atomes de carbone, linéaires ou ramifiés, les halogènes et les groupements hydroxyle, amino, nitro et alcoxy ayant de 1 à 10 atomes de carbone ; et OSiRR'R" où R, R' et R" sont choisis indépendamment les uns des autres parmi les groupements alkyle ayant de 1 à 10 atomes de carbone, linéaires, cycliques ou ramifiés, les groupements aryle ayant de 6 à 10 atomes de carbone, éventuellement substitués par un ou des substituants choisis parmi les groupements alkyle ayant de 1 à 10 atomes de carbone, linéaires ou ramifiés, ou R1 et R2 représentent ensemble =O,
R3 représente COOH ou un groupement COOR5 où R5 représente un groupement choisi parmi les groupements alkyle ayant de 1 à 10 atomes de carbone, linéaires, cycliques ou ramifiés, benzyle, et les groupements benzyle substitués par un ou deux substituants choisis parmi les groupements alkyle ayant de 1 à 10 atomes de carbone, linéaires ou ramifiés, les halogènes et les groupements hydroxyle, amino, nitro et alcoxy ayant de 1 à 10 atomes de carbone, ou R3 représente un groupe cyano,
R6 et R7 sont choisis indépendamment les uns des autres parmi H, les groupements alkyle ayant de 1 à 10 atomes de carbone, linéaires, cycliques ou ramifiés, et les groupements aryle ayant de 6 à 10 atomes de carbone, éventuellement substitués par un ou des substituants choisis parmi les groupements alkyle ayant de 1 à 10 atomes de carbone, linéaires ou ramifiés, les halogènes et les groupements hydroxyle, amino, nitro et alcoxy ayant de 1 à 10 atomes de carbone ; R8 est choisi parmi H, les groupements alkyle ayant de 1 à 10 atomes de carbone, linéaires, cycliques ou ramifiés, les groupes aryle ayant de 6 à 10 atomes de carbone, éventuellement substitués par un ou des substituants choisis parmi les groupements alkyle ayant de 1 à 10 atomes de carbone, linéaires ou ramifiés, et les groupements attracteurs notamment ceux comprenant une fonction choisie parmi les fonctions acide, ester, cyano, et X représente un contre-ion,
pour préparer de la 2-hydroxybutyrolactone.

## Patentansprüche

1. Verfahren zur Herstellung von 2-Hydroxybutyrolacton (2HBL) aus einer Verbindung oder ihrem Salz oder ihren Oligomeren, wobei die besagte Verbindung der Formel (I) entspricht:
CH₃-S-CH₂CH₂CR1R2R3,
in der
R1 für H steht,
R2 für eine Gruppe steht, die aus OH; OR4 und OCOR4, wobei R4 für eine Gruppe steht, die aus linearen, cyclischen oder verzweigten Alkylgruppen mit 1 bis 10 Kohlenstoffatomen und Arylgruppen mit 6 bis 10 Kohlenstoffatomen, die gegebenenfalls durch einen oder mehrere Substituenten substituiert sind, der bzw. die aus linearen oder verzweigten Alkylgruppen mit 1 bis 10 Kohlenstoffatomen, Halogenen und Hydroxyl-, Amino-, Nitro- und Alkoxygruppen mit 1 bis 10 Kohlenstoffatomen ausgewählt ist bzw. sind, ausgewählt ist; und OSiRR'R" ausgewählt ist, wobei R, R' und R" unabhängig voneinander aus linearen, cyclischen oder verzweigten Alkylgruppen mit 1 bis 10 Kohlenstoffatomen, Arylgruppen mit 6 bis 10 Kohlenstoffatomen, die gegebenenfalls durch einen oder mehrere Substituenten substituiert sind, der bzw. die aus linearen oder verzweigten Alkylgruppen mit 1 bis 10 Kohlenstoffatomen ausgewählt ist bzw. sind, ausgewählt sind, oder R1 und R2 zusammen für =O stehen,
R3 für COOH oder eine COOR5-Gruppe steht, wobei R5 für eine Gruppe steht, die aus linearen, cyclischen oder verzweigten Alkylgruppen mit 1 bis 10 Kohlenstoffatomen, Benzyl und Benzylgruppen, die durch einen oder zwei Substituenten substituiert sind, der bzw. die aus linearen oder verzweigten Alkylgruppen mit 1 bis 10 Kohlenstoffatomen, Halogenen und Hydroxyl-, Amino-, Nitro- und Alkoxygruppen mit 1 bis 10 Kohlenstoffatomen ausgewählt ist bzw. sind, ausgewählt ist, oder R3 für eine Cyanogruppe steht,
ein Verfahren, nach dem
ein Sulfonium der besagten Verbindung, wobei das besagte Sulfonium der Formel (II) entspricht:
[CH₃][CH₂CH₂CR1R2R3][CR6R7R8]S⁺X⁻,
durch Reaktion mit einem Agens der Formel [CR6R7R8]X oder der Formel [CR6R7R8]⁺X⁻ erhalten wird,
Formeln, in denen R1, R2 und R3 die obige Definition haben und R6 und R7 unabhängig voneinander aus H, linearen, cyclischen oder verzweigten Alkylgruppen mit 1 bis 10 Kohlenstoffatomen und Arylgruppen mit 6 bis 10 Kohlenstoffatomen, die gegebenenfalls durch einen oder mehrere Substituenten substituiert sind, der bzw. die aus linearen oder verzweigten Alkylgruppen mit 1 bis 10 Kohlenstoffatomen, Halogenen und Hydroxyl-, Amino-, Nitro- und Alkoxygruppen mit 1 bis 10 Kohlenstoffatomen ausgewählt ist bzw. sind, ausgewählt sind; R8 aus H, linearen, cyclischen oder verzweigten Alkylgruppen mit 1 bis 10 Kohlenstoffatomen, Arylgruppen mit 6 bis 10 Kohlenstoffatomen, die gegebenenfalls durch einen oder mehrere Substituenten substituiert sind, der bzw. die aus linearen oder verzweigten Alkylgruppen mit 1 bis 10 Kohlenstoffatomen ausgewählt ist bzw. sind, und Attraktorgruppen, insbesondere jenen, die eine Funktion umfassen, die aus Säure-, Ester-, Cyanofunktionen ausgewählt sind, ausgewählt ist, und X für ein Gegenion steht, und
das so erhaltene Sulfonium zu 2,4-Dihydroxybuttersäure oder ihr Salz hydrolysiert wird und
die 2,4-Dihydroxybuttersäure oder ihr Salz zu 2-Hydroxybutyrolacton cyclisiert wird.

2. Verfahren nach Anspruch 1, **dadurch gekennzeichnet, dass** die Verbindung der Formel (I) aus 2-Hydroxy-4-methylthiobuttersäure (HMTB), 2-Oxo-4-methylthiobuttersäure (KMB), Isopropylester von HMTB (HMBI), ihren Salzen oder ihren Oligomeren ausgewählt ist.

3. Verfahren nach Anspruch 1 oder 2, **dadurch gekennzeichnet, dass** R2 für OH steht, R6 und R7 unabhängig aus H und CH₃ ausgewählt sind und R8 aus H und CH₃-, Phenyl- und COOH-Gruppen ausgewählt ist.

4. Verfahren nach einem der Ansprüche 1 bis 3, **dadurch gekennzeichnet, dass** das Sulfonium durch Reaktion auf der Verbindung mit einem Agens erhalten wird, das aus den Agentien der Formel [CR6R7R8]X ausgewählt ist, wobei X aus Halogenen und OH-, Sulfat-, Sulfonat- und Phosphatgruppen ausgewählt ist.

5. Verfahren nach Anspruch 4, **dadurch gekennzeichnet, dass** das besagte Agens aus Bromessigsäure und Benzylbromid ausgewählt ist.

6. Verfahren nach Anspruch 4, **dadurch gekennzeichnet, dass** das besagte Agens aus linearen oder verzweigten Alkoholen mit 2 bis 6 Kohlenstoffatomen ausgewählt ist und in einem sauren Medium verwendet wird.

7. Verfahren nach Anspruch 1, **dadurch gekennzeichnet, dass** das Sulfonium durch Reaktion auf der Verbindung mit einem Agens der Formel [CR6R7R8]⁺X⁻ erhalten wird, wobei [CR6R7R8]⁺ ein Carbokation ist, das aus einem entsprechenden linearen oder verzweigten Alken mit 2 bis 10 Kohlenstoffatomen in Gegenwart einer Säure gebildet wird.

8. Verfahren nach Anspruch 7, **dadurch gekennzeichnet, dass** das Agens die Formel [C(CH₃)₂H]⁺X⁻ hat, wobei X für HSO₄ oder Cl steht, und aus Propen in Gegenwart von Schwefelsäure bzw. Salzsäure gebildet wird.

9. Verfahren nach Anspruch 7, **dadurch gekennzeichnet, dass** das Agens die Formel [C(CH₃)₃]⁺X⁻ hat, wobei X für HSO₄ oder Cl steht, und aus Isobuten in Gegenwart von Schwefelsäure bzw. Salzsäure gebildet wird.

10. Verfahren nach Anspruch 1, **dadurch gekennzeichnet, dass** mindestens zwei der Reaktionen zur Bildung des Sulfoniums, zur Hydrolyse des Sulfoniums und zur Cyclisierung der 2,4-Dihydroxybuttersäure (24DHBA) simultan ablaufen.

11. Verfahren nach Anspruch 10, **dadurch gekennzeichnet, dass** die Reaktionstemperatur von 30 bis 150 °C variiert.

12. Verfahren nach Anspruch 10 oder 11, **dadurch gekennzeichnet, dass** Halogenidsalze wie NaBr zugegeben werden.

13. Verfahren nach einem der Ansprüche 1 bis 12, **dadurch gekennzeichnet, dass** es im industriellen Maßstab durchgeführt wird.

14. Verwendung eines Sulfoniums, das der Formel (II) entspricht:
[CH₃][CH₂CH₂CR1R2R3][CR6R7R8]S⁺X⁻,
in der
R1 für H steht,
R2 für eine Gruppe steht, die aus OH; OR4 und OCOR4, wobei R4 für eine Gruppe steht, die aus linearen, cyclischen oder verzweigten Alkylgruppen mit 1 bis 10 Kohlenstoffatomen und Arylgruppen mit 6 bis 10 Kohlenstoffatomen, die gegebenenfalls durch einen oder mehrere Substituenten substituiert sind, der bzw. die aus linearen oder verzweigten Alkylgruppen mit 1 bis 10 Kohlenstoffatomen, Halogenen und Hydroxyl-, Amino-, Nitro- und Alkoxygruppen mit 1 bis 10 Kohlenstoffatomen ausgewählt ist bzw. sind, ausgewählt ist; und OSiRR'R" ausgewählt ist, wobei R, R' und R" unabhängig voneinander aus linearen, cyclischen oder verzweigten Alkylgruppen mit 1 bis 10 Kohlenstoffatomen, Arylgruppen mit 6 bis 10 Kohlenstoffatomen, die gegebenenfalls durch einen oder mehrere Substituenten substituiert sind, der bzw. die aus linearen oder verzweigten Alkylgruppen mit 1 bis 10 Kohlenstoffatomen ausgewählt ist bzw. sind, ausgewählt sind, oder R1 und R2 zusammen für =O stehen,
R3 für COOH oder eine COOR5-Gruppe steht, wobei R5 für eine Gruppe steht, die aus linearen, cyclischen oder verzweigten Alkylgruppen mit 1 bis 10 Kohlenstoffatomen, Benzyl und Benzylgruppen, die durch einen oder zwei Substituenten substituiert sind, der bzw. die aus linearen oder verzweigten Alkylgruppen mit 1 bis 10 Kohlenstoffatomen, Halogenen und Hydroxyl-, Amino-, Nitro- und Alkoxygruppen mit 1 bis 10 Kohlenstoffatomen ausgewählt ist bzw. sind, ausgewählt ist, oder R3 für eine Cyanogruppe steht,
R6 und R7 unabhängig voneinander aus H, linearen, cyclischen oder verzweigten Alkylgruppen mit 1 bis 10 Kohlenstoffatomen und Arylgruppen mit 6 bis 10 Kohlenstoffatomen, die gegebenenfalls durch einen oder mehrere Substituenten substituiert sind, der bzw. die aus linearen oder verzweigten Alkylgruppen mit 1 bis 10 Kohlenstoffatomen, Halogenen und Hydroxyl-, Amino-, Nitro- und Alkoxygruppen mit 1 bis 10 Kohlenstoffatomen ausgewählt ist bzw. sind, ausgewählt sind; R8 aus H, linearen, cyclischen oder verzweigten Alkylgruppen mit 1 bis 10 Kohlenstoffatomen, Arylgruppen mit 6 bis 10 Kohlenstoffatomen, die gegebenenfalls durch einen oder mehrere Substituenten substituiert sind, der bzw. die aus linearen oder verzweigten Alkylgruppen mit 1 bis 10 Kohlenstoffatomen ausgewählt ist bzw. sind, und Attraktorgruppen, insbesondere jenen, die eine Funktion umfassen, die aus Säure-, Ester-, Cyanofunktionen ausgewählt sind, ausgewählt ist, und X für ein Gegenion steht,
zum Herstellen von 2-Hydroxybutyrolacton.

## Claims

1. A method for preparing 2-hydroxybutyrolactone (2HBL) from a compound, or from its salt or its oligomers, said compound fitting formula (I)
CH₃-S-CH₂CH₂CR1R2R3
wherein
R1 represents H
R2 represents a group selected from OH; OR4 and OCOR4 wherein R4 represents a group selected from linear, cyclic or branched alkyl groups having from 1 to 10 carbon atoms, and aryl groups having from 6 to 10 carbon atoms, optionally substituted with substituent(s) selected from linear or branched alkyl groups having from 1 to 10 carbon atoms halogens and hydroxyl, amino, nitro and alkoxy groups having from 1 to 10 carbon atoms; and OSiRR'R" wherein R, R' and R" are selected independently of each other from linear, cyclic or branched alkyl groups having from 1 to 10 carbon atoms, aryl groups having from 6 to 10 carbon atoms, optionally substituted with substituent(s) selected from linear or branched alkyl groups having from 1 to 10 carbon atoms, or R1 and R2 represent together =O,
R3 represents COOH or a COOR5 wherein R5 represents a group selected from linear, cyclic or branched alkyl groups having from 1 to 10 carbon atoms, benzyl groups and benzyl groups substituted with one or two substituents selected from linear or branched alkyl groups having from 1 to 10 carbon atoms, halogens and hydroxyl, amino, nitro and alkoxy groups having from 1 to 10 carbon atoms, or R3 represents a cyano group,
method according to which
a sulfonium of said compound is obtained, said sulfonium fitting the formula (II)
[CH₃][CH₂CH₂CR1R2CR3][CR6R7R8]S⁺ X⁻
By reacting with an agent fitting formula [CR6R7R8]X
wherein R1, R2 and R3 have the above definition, and R6 and R7 are selected independently of each other from H, linear, cyclic or branched alkyl groups having from 1 to 10 carbon atoms, and aryl groups having from 6 to 10 carbon atoms optionally substituted with substituent(s) selected from linear or branched alkyl groups having from 1 to 10 carbon atoms, halides and hydroxyl, amino, nitro and alkoxy groups having from 1 to 10 carbon atoms; R8 is selected from H, linear, cyclic or branched alkyl groups having from 1 to 10 carbon atoms, aryl groups having from 6 to 10 carbon atoms, optionally substituted with substituent(s) selected from linear or branched alkyl groups having from 1 to 10 carbon atoms, and attractor groups notably those comprising a function selected from acid, ester, cyano functions and X represents a counter-ion, and
the thereby obtained sulfonium is hydrolyzed in 2,4-dihydroxybutyric acid or its salt, and
2,4-dihydroxybutyric acid or its salt is cyclized into 2-hydroxybutyrolactone.

2. The method according to claim 1, **characterized in that** the compound of formula (I) is selected from 2-hydroxy-4-methylthiobutyric acid (HMTB), 2-oxo-4-methylthiobutyric acid (KMB), HMTB isopropyl ester (HMBI), salts thereof and oligomers thereof.

3. The method according to claim 1 or 2, **characterized in that** R2 represents OH, R6 and R7 are independently selected from H and CH₃ and R8 is selected from H and CH₃, phenyl and COOH groups.

4. The method according to any of claims 1 to 3, **characterized in that** the sulfonium is obtained by reaction on the compound, of an agent selected from agents of formula [CR6R7R8]X wherein X is selected from halogens and OH, sulfate, sulfonate and phosphate groups.

5. The method according to claim 4, **characterized in that** said agent is selected from bromoacetic acid and benzyl bromide.

6. The method according to claim 4, **characterized in that** said agent is selected from linear or branched alcohols, having from 2 to 6 carbon atoms and is used in an acid medium.

7. The method according to claim 1, **characterized in that** sulfonium is obtained by reaction on the compound of an agent of formula [CR6R7R8]⁺X⁻ wherein [CR6R7R8]⁺ is a carbocation formed from a corresponding linear or branched alkene having from 2 to 10 carbon atoms, in the presence of an acid.

8. The method according to claim 7, **characterized in that** the agent is of formula [C(CH₃)₂H]⁺X⁻ wherein X represents HSO₄ or Cl and is formed from propene in the presence of sulfuric acid or hydrochloric acid respectively.

9. The method according to claim 7, **characterized in that** the agent is of formula [C(CH₃)₃]⁺X⁻ wherein X represents HSO₄ or Cl and is formed from isobutene in the presence of sulfuric acid or hydrochloric acid respectively.

10. The method according to claim 1, **characterized in that** at least two of the reactions for forming the sulfonium, for hydrolyzing the sulfonium and for cyclizing 2,4-dihydroxybutyric acid (24DHBA) are simultaneous.

11. The method according to claim 10, **characterized in that** the reaction temperature varies from 30 to 150°C.

12. The method according to claim 10 or 11, **characterized in that** halide salts such as NaBr are added.

13. The method according to any of claims 1 to 12, **characterized in that** it is applied on an industrial scale.

14. The use of a sulfonium fitting formula (II)
[CH₃][CH₂CH₂CR1R2CR3][CR6R7R8]S⁺ X⁻
wherein
R1 represents H
R2 represents a group selected from OH; OR4 and OCOR4 wherein R4 represents a group selected from linear, cyclic or branched alkyl groups having from 1 to 10 carbon atoms and aryl groups having from 6 to 10 carbon atoms optionally substituted with substituent(s) selected from linear or branched alkyl groups having from 1 to 10 carbon atoms, halogens and hydroxyl, amino, nitro and alkoxy groups having from 1 to 10 carbon atoms; and OSiRR'R" wherein R, R' and R" are selected independently of each other from linear, cyclic or branched alkyl groups having from 1 to 10 carbon atoms, aryl groups having from 6 to 10 carbon atoms, optionally substituted with substituent(s) selected from linear or branched alkyl groups having from 1 to 10 carbon atoms, or R1 and R2 represent together =O,
R3 represents COOH or a COOR5 groups wherein R5 represents a group selected from linear, cyclic or branched alkyl groups having from 1 to 10 carbon atoms, benzyl groups, and benzyl groups substituted with one or two substituents selected from linear or branched alkyl groups having from 1 to 10 carbon atoms, halogens and hydroxyl, amino, nitro and alkoxy groups having from 1 to 10 carbon atoms, or R3 represents a cyano group,
R6 and R7 are selected independently of each other from H, linear, cyclic or branched alkyl groups having from 1 to 10 carbon atoms, and aryl groups having from 6 to 10 carbon atoms, optionally substituted with substituent(s) selected from linear or branched alkyl groups having from 1 to 10 carbon atoms, halides and hydroxyl, amino, nitro and alkoxy groups having from 1 to 10 carbon atoms; R8 is selected from H, linear, cyclic or branched alkyl groups having from 1 to 10 carbon atoms, aryl groups having from 6 to 10 carbon atoms, optionally substituted with substituent(s) selected from linear or branched alkyl groups having from 1 to 10 carbon atoms, and attractor groups notably those comprising a function selected from acid, ester, cyano functions and X represents a counter-ion and
for preparing 2-hydroxybutyrolactone.
